(19) Europäisches Patentamt
European Patent Office
Office européen des brevets

(11) Veröffentlichungsnummer: **0 271 812**
**B1**

(12) # EUROPÄISCHE PATENTSCHRIFT

(45) Veröffentlichungstag der Patentschrift: **28.02.90**

(51) Int. Cl.⁴: **C07C 47/127**, C07C 45/37

(21) Anmeldenummer: **87118194.7**

(22) Anmeldetag: **09.12.87**

(54) **Verfahren zur Herstellung von Carbonylverbindungen.**

(30) Priorität: **19.12.86 DE 3643469**

(43) Veröffentlichungstag der Anmeldung:
**22.06.88 Patentblatt 88/25**

(45) Bekanntmachung des Hinweises auf die Patenterteilung:
**28.02.90 Patentblatt 90/9**

(84) Benannte Vertragsstaaten:
**BE CH DE FR GB IT LI**

(56) Entgegenhaltungen:
**EP-A- 0 007 570**
**DE-A- 1 923 048**
**DE-C- 1 967 147**
**US-A- 4 258 216**
**US-A- 4 503 261**
**US-A- 4 555 583**

**Patent Abstracts of Japan, unexamined applications, C Field, Vol. 9, Nr. 241, 27. September 1985 The Patent Office Japanese Government Seite 97 C 306**

(73) Patentinhaber: **BASF Aktiengesellschaft, Carl-Bosch-Strasse 38, D-6700 Ludwigshafen(DE)**

(72) Erfinder: **Graf, Fritz, Dr., Im Rothschild 33, D-6720 Speyer(DE)**
Erfinder: **Engelbach, Heinz, Dr., Kropsburgstrasse 24, D-6703 Limburgerhof(DE)**
Erfinder: **Hupfer, Leopold, Dr., Waltershoehe 3, D-6701 Friedelsheim(DE)**
Erfinder: **Schultheiss, Harald, Dr., An der Adamslust 9, D-6710 Frankenthal(DE)**
Erfinder: **Sprague, Michael Jolyon, Dr., Lachner Strasse 3, D-6800 Mannheim 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein neues Verfahren zur Herstellung von Carbonylverbindungen durch Gasphasenoxidation von Alkoholen mit einem Sauerstoff enthaltenden Gas in Gegenwart von Kupfer und/oder Silber enthaltenden Katalysatoren.

Aus der DE-PS 19 23 048 ist bekannt, daß man Glyoxal durch Gasphasenoxidation von Ethylenglykol an einem Oxidationskatalysator herstellen kann, der Kupfer in Kombination mit Zinn, Phosphor, Arsen, Antimon oder Wismut enthält. Der Katalysator, der zusätzlich auch Silber enthalten kann, wird als Legierung eingesetzt, vorzugsweise in Form von Drehspänen oder Gaze. Für die Kombination Silber/Kupfer/Phosphor werden Ausbeuten -bezogen auf umgesetztes Ethylenglykol - zwischen 55 % und 72 % angegeben. Die höheren Werte können allerdings nur unter Inkaufnahme eines unvollständigen Glykolumsatzes erzielt werden. Dies ist in wirtschaftlicher Sicht unbefriedigend, da eine Entfernung des restlichen Glykols aus dem Produkt nur mit großem Aufwand möglich ist. Außerdem werden nach diesem bekannten Verfahren unzureichende Raum-Zeit-Ausbeuten erhalten. Nachteilig ist ferner die umständliche Katalysatorherstellung.

In der DE-PS 19 67 147 wird für die Herstellung von Glyoxal aus Ethylenglykol ein Silberkatalysator vorgeschlagen, der Phosphor enthält. Auch bei diesem Verfahren muß eine Glyoxalausbeute bis 70 %, bezogen auf umgesetztes Ethylenglykol, mit unbefriedigenden Umsätzen erkauft werden. Die Raum-Zeit-Ausbeuten sind ebenfalls unbefriedigend.

Nach den Angaben der DE-OS 21 58 343 sinkt die Selektivität von in der DE-PS 19 23 048 beschriebenen Katalysatoren bei einer 700-stündigen Betriebsdauer von einem Anfangswert von 60 bis 64 % auf 55 % ab. Es ist ferner angegeben, daß man einen Kupfer, Silber und Phosphor enthaltenden Katalysator nach einer 700-stündigen Laufzeit durch eine 12-stündige Reduktion bei 450°C regenerieren kann. In der DE-OS 21 58 344 wird die Regenerierung eines Kupfer und Phosphor enthaltenden Katalysators beschrieben, bei der über den Katalysator mindestens 1 Tag Sauerstoffüberschuß geleitet wird. Diese Regenerierverfahren haben den Nachteil, daß sie mit einem Produktionsausfall verbunden sind und aufwendige Sicherheitsmaßnahmen erfordern, um die Vermischung der zum Regenerieren verwendeten Luft mit dem Reaktionsgas auszuschließen.

Nach den Verfahren der US-PS 4 282 374 und der US-PS 4 503 261 erhält man bei der Gasphasenoxidation von Ethylenglykol an Kupferkatalysatoren oder an einem Schichtkatalysator aus Kupfer- und Silberkristallen vorteilhafte Ergebnisse hinsichtlich der Lebensdauer der Katalysatoren und der Glyoxalausbeute, wenn man die Reaktion in Gegenwart einer flüchtigen Phosphorverbindung durchführt, wobei die Phosphormenge (berechnet P), bezogen auf die Gewichtsmenge an Ethylenglykol, 1 bis 100 ppm bzw. 0,5 bis 20 ppm beträgt. Bei diesen Verfahren hat sich jedoch bei längerer Betriebsdauer herausgestellt, daß die Glyoxalausbeute und Produktreinheit mit der Versuchsdauer zunehmend schlechter werden. Dieser Nachteil ist auf eine vermehrte Bildung von Fromaldehyd und von $CO/CO_2$ zurückzuführen.

Anteile von Formaldehyd im Glyoxal sind für viele Anwendungszwecke des Glyoxals wegen der toxikologischen Eigenschaften und der hohen Reaktivität des Formaldehyds höchst unerwünscht. Da man Formaldehyd nur mit einem erheblichen Aufwand und unter Inkaufnahme von Ausbeuteverlusten aus dem rohen Glyoxal entfernen kann, etwa durch Behandlung mit Wasserdampf oder durch chemische Umsetzung, war nach einem Verfahren zu suchen, das es gestattet, Glyoxal durch katalytische Gasphasenoxidation von Ethylenglykol auch bei langen Betriebszeiten unter weitgehender Vermeidung der Bildung störender Nebenprodukte herzustellen.

Es wurde nun gefunden, daß man bei der Herstellung von Carbonylverindungen der Formel

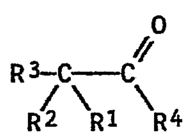 I,

in der $R^1$ ein Wasserstoffatom oder zusammen mit $R^2$ ein Sauerstoffatom, $R^2$ den Rest $OR^5$ oder zusammen mit $R^1$ ein Sauerstoffatom, $R^3$ ein Wasserstoffatom, einen Alkylrest mit 1 bis 8 C-Atomen oder einen Cyclohexyl- oder Cyclopentylrest, $R^4$ ein Wasserstoffatom oder einen Alkylrest mit 1 bis 3 C-Atomen und $R^5$ einen Alkylrest mit 1 bis 4 C-Atomen, einen Cyclohexyl- oder Cyclopentylrest oder einen Rest der Formeln $-CH_2-CH$ oder $-CH_2-CH_2-O-CH_2-CHO$ bedeuten, durch Gasphasenoxidation von Alkoholen der Formel

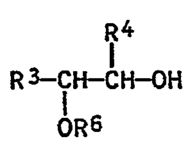 II,

in der $R^3$ und $R^4$ die oben angegebene Bedeuteun haben und $R^6$ ein Wasserstoffatom, einen Alkylrest mit 1 bis 4 C-Atomen, einen Cyclohexyl- oder Pentylrest oder einen Rest der Formeln $-CH_2-CH_2-OH$ oder $-CH_2-CH_2-O-CH_2-CH_2-OH$ bedeutet, mit einem Sauerstoff enthaltenden Gas in Gegenwart von Kupfer oder Silber entahltenden Katalysatoren und einer unter den Reaktionsbedingungen flüchtigen Phosphorverbindung die Carbonylverbindungen auf besonders vorteilhafte Weise herstellen kann, wenn man die Menge der Phosphorverbindung so bemißt, daß sie, bezogen auf die Gewichtsmenge an den eingesetzten Alkohol, geringer ist als 0,5 ppm.

Nach dem neuen Verfahren, bei dem man die Phosphormenge (berechnet P), bezogen auf die Gewichtsmeng an dem Alkohol, auf Werte unter 0,5 ppm beschränkt, erhält man z.B. das Glyoxal aus Ethylenglykol auch im Dauerbetrieb in hoher Ausbeute und Reinheit. Dieses vorteilhafte Ergebnis ist überraschend, da man nach den Angaben in der US-PS 4 503 261 (Spalte 3, Zeilen 54 - 61) eine Phosphormenge (berechnet P) von 0,5 bis 20 ppm, bezogen auf das Gewicht an Ethylenglykol, für erfoderlich gehalten hat, um einerseits die Bildung von $CO_2$ zurückzudrängen und um andererseits aber nicht die Aktivität des Katalysators zu beeinträchtigen.

In den Alkoholen der Formel II bedeuten Alkylreste z.B. Methyl-, Ethyl-. Propyl- oder Butylreste. Bei dem erfingungsgemäßen Verfahren gehen die endständigen Hydroxylgruppen in Aldehydgruppen und die sekundären Hydroxylgruppen in Ketogruppen über.

Beispielsweise seien die folgenden Ausgangsverbindungen der Formel II genannt:

$HO-CH_2-CH_2-OH$, $H_3COCH_2-CH_2OH$, $C_2H_5OCH_2-CH_2OH$,

$H_3C-CH(OH)-CH_2-OH$, $C_2H_5-CH(OH)-CH_2OH$, 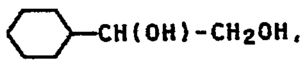,

$HO-(CH_2)_2-O-(CH_2)_2-OH$, 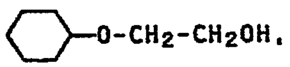, 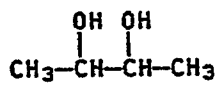

Die Gasphasenoxidation des Alkohols mit dem Sauerstoff enthaltenden Gas an den Kupfer und/oder Silber enthaltenden Katalysatoren führt man auf an sich bekannte Weise, z.B. bie Temperaturen von ca. 225 bis ca 500°C, durch. Als Kupfer oder Silber enthaltende Katalysatoren sind beispielsweise metallisches Kupfer oder Silber, kupferhaltige oder siilberhaltige Legierungen oder Verbindungen mit Metallen oder Nichtmetallen geeignet, wie Kupferphosphide, Kupferbronzen oder Legierungen des Kupfers mit Silber und/oder Gold, Kupfererze wie Malachit und Kupfer- oder Silberverbindungen, die während der Reaktion ganz oder teilweise zu Kupfer oder Silber reduziert werden können, wie Kupfer(I)oxid, Silber(I)oxid, Kupfer(II)oxid, und Verbindungen, die beim Erhitzen in Kupferoxide übergehen, wie Kupfernitrat und Kupferazetat. Ferner sind auch Kupferphosphat und Kupferantimonat geeignet. Den kupferhaltigen Verbindungen können zusätzlich noch andere Metall- oder Nichtmetalloxide wie die Oxide von Zink, Chrom, Phosphor, Antimon, Zinn und Wismut beigemischt sein. Die kupfer oder silberhaltige katalytische Masse kann auch auf einem inerten Träger aufgebracht oder gegebenenfalls mit einem inerten Material verdünnt werden. Der Katalysator kann gegebenenfalls auch vor dem Einsatz einer reduzierenden Behandlung unterworfen werden.

Bevorzugt sind Katalysatoren, die keine große innere Oberfläche besitzen, beispielsweise solche mit einer Oberfläche von unter 50 $m^2$ pro g. Besonderes technisches Interesse haben metallisches Kupfer oder Silber und Legierungen, die Kupfer oder Silber als einen wesentlichen Bestandteil enthalten. Man wendet sie z.B. in Form von Drehspänen, Drahtgeweben, Gazen oder auch als Trägerkatalysatoren mit einem z.B. oberflächenarmen, inerten Träger an.

Als unter den Reaktionsbedingungen flüchtige Phosphorverbindungen verwendet man zweckmäßigerweise P-Verbindungen, die unzersetzbar verdampfbar sind und mit den Komponenten des Synthesegases bei den Umsetzungsbedingungen keine Reaktion eingehen. Das sind z.B. Ester der Phosphorsäure, der phosphorigen Säure oder von Phosphonsäuren, wie Trimethylphosphat, Triethylphosphat, Tri-isopropylphosphat, Tri-n-propylphosphat, Trimethylphosphit, Triethylphosphit, Triethylphosphinoxid, Methylphosphonsäurediethylester, Methylphosphonsäuredimethylester oder Ethylphosphonsäurediethylester. Erfindungsgemäß werden die Phosphorverbindungen so eingesetzt, daß die zugegebene Phosphormenge (berechnet P), bezogen auf die Gewichtsmenge an dem Alkohol, geringer ist als 0,5 ppm. Vorzugsweise liegt sie im Bereich von 0,05 bis 0,48 ppm.

Das Verfahren wird z.B. so ausgeführt, daß man ein gasförmiges Gemisch aus dem Alkohol und Wasser (Wassergehalt 0,1 bis 99 %) zusammen mit Sauerstoff in einer Menge zwischen ca. 0,5 und 2,0 mol, bezogen auf 1 mol des eingesetzten Alkohols, eventuell zusammen mit Stickstoff (0 bis 99 Vol.% des Gesamtgasgemisches) über den auf 225 bis 500°C gehaltenen Katalysator leitet, wobei man die flüchtige Phosphorverbindung dem gasförmigen Ausgangsgemisch vorher zusetzt. Das den Reaktor verlassende gasförmige Reaktionsgemisch wird wie üblich mit Wasser ausgewaschen. Zur besseren Kontrolle der zugegebenen Phosphormenge im Spurenbereich kann man zweckmäßigerweise auch so vorgehen, daß

man die Phosphorverbindung in Wasser oder dem eingesetzten Alkohol gelöst in den heißen Synthesegasstrom eindosiert.

Das nach dem erfindungsgemäßen Verfahren aus Ethylenglykol erhaltene Glyoxal, das direkt in der handelsüblichen Form einer 40%igen wäßrigen Lösung erhalten werden kann, zeichnet sich durch eine hohe Reinheit aus, die auch bei langem Betriebszeitraum unverändert bleibt. So liegt der Restgehalt der 40%igen Glyoxallösung an unumgesetztem Glykol unter 0,2 %. Der Gehalt an Glykolaldehyd beträgt etwa 0,1 % und der Formaldehydgehalt liegt bei 0,5 %.

Beispiel 1

123 Teile Kupferformkörper werden in einem Rohrreaktor aus rostfreiem Stahl mit einem inneren Durchmesser von 20 mm so eingebaut, daß die Katalysatorfüllhöhe 30 cm beträgt. Man leitet pro Stunde ein Synthesegasgemisch aus 12 Gew.-Teilen Ethylenglykol, 27 Vol.-Teilen Luft und 100 Vol.-Teilen Stickstoff durch den Rohrreaktor. Zum Synthesegas werden 0,3 ppm P, bezogen auf die eingesetzte Gewichtsmenge an Ethylenglykol, in Form von Trimethylphosphat zugegeben. Die Reaktion wird bei einer Temperatur von 360°C durchgeführt. Das Reaktionsgas wird nach Verlassen des Reaktors mit Wasser in Berührung gebracht und die Reaktionsprodukte in der wäßrigen Phase gelöst.

Man erhält eine Glyoxalausbeute von 77,1 Mol%, bezogen auf das eingesetzte Ethylenglykol. Formaldehyd entsteht mit einer Ausbeute von 0,6 Mol%. Die Verbrennung zu CO und $CO_2$ beträgt 15,5 %. Als weiteres Nebenprodukt entsteht Glykolaldehyd mit einer Ausbeute von 1,0 Mol%. Nach einer Laufzeit von 15 Tagen liegt die Glyoxalausbeute bei 79,3 %. Die Ausbeuten an Formaldehyd, CO/$CO_2$ und Glykolaldehyd werden zu 0,5 Mol%, 16,3 Mol% und 0,5 Mol% bestimmt.

Beispiel 2

Man verfährt wie in Beispiel 1 beschrieben, wobei die Katalysatormasse 121 Gew.-Teile beträgt und das Synthesegas aus 12 Gew.-Teilen Ethylenglykol, 28,5 Vol.-Teilen Luft und 100 Vol.-Teilen Stickstoff besteht. Man dosiert soviel Trimethylphosphat zum Synthesegasgemisch, daß die Phosphormenge 0,1 ppm, bezogen auf das eingesetzte Ethylenglykol, beträgt.

Man erhält eine Glyoxalausbeute von 72,7 Mol%, bezogen auf das eingesetzte Ethylenglykol. Formaldehyd entsteht mit einer Ausbeute von 0,7 Mol%. Die Verbrennung zu CO und $CO_2$ beträgt 14,4 %. Als weiteres Nebenprodukt entsteht Glykolaldehyd mit einer Ausbeute von 1,8 Mol%. Nach einer Laufzeit von 10 Tagen liegt die Glyoxalausbeute bei 73,6 %. Die Ausbeuten an Formaldehyd, CO/$CO_2$ und Glykolaldehyd betragen 0,6 Mol%, 15,3 Mol% und 2,3 Mol%.

Beispiel 3

14,5 Teile eines Katalysators aus silberbeschichteten Steatitkugeln mit einem Durchmesser von 3 mm werden in einem Rohrreaktor aus rostfreiem Stahl mit einem Durchmesser von 20 mm so eingebaut, daß die Katalysatorfüllhöhe 30 cm beträgt. Man leitet pro Stunde ein Synthesegasgemisch aus 6 Gew.-Teilen Ethylenglykol, 13,5 Vol.-Teilen Luft und 100 Vol.-Teilen Stickstoff durch den Rohrreaktor. Zum Synthesegas werden 0,4 ppm P, bezogen auf die eingesetzte Gewichtsmenge an Ethylenglykol, in Form von Trimethylphosphat zugegeben. Die Reaktionstemperatur beträgt 360°C. Das Reaktionsgas wird wie in Beispiel 1 beschrieben aufgearbeitet.

Man erhält eine Glyoxalausbeute von 67 Mol.%, bezogen auf das eingesetzte Ethylenglykol. Formaldehyd, CO und $CO_2$ sowie Glykolaldehyd entstehen mit Ausbeuten von 1,0, 21,4 sowie 2,7 Mol.%. Nach 7 Tagen Laufzeit beträgt die Glyoxalausbeute 70 Mol.% bei Ausbeuten von 0,9 Mol.% Formaldehyd, 22,9 Mol.% CO und $CO_2$ sowie 1,5 Mol.% Glykolaldehyd.

Beispiel 4 (Vergleich)

Man verfährt in der in Beispiel 1 beschriebenen Weise, wobei die Katalysatormasse 115 Gew.-Teile beträgt. Das Synthesegas besteht aus 11,8 Gew.-Teilen Ethylenglykol, 27 Vol.-Teilen Luft sowie 96,4 Vol.-Teilen Stickstoff. Man dosiert soviel Trimethylphosphat zum Synthesegas, daß die Phosphormenge 1,5 ppm, bezogen auf das eingesetzte Ethylenglykol, beträgt.

Man erhält eine Glyoxalausbeute von 74 Mol%, bezogen auf das eingesetzte Ethylenglykol. Formaldehyd entsteht mit einer Ausbeute von 1,0 Mol%. Die Bildung von $CO_x$ erfolgt mit 18 Mol%. Nach einer Laufzeit von 30 Tagen liegt die Glyoxalausbeute bei 71 %. Die Ausbeute an Formaldehyd und $CO_x$ wird zu 4,3 und 18,5 Mol% bestimmt. Nach einer weiteren Laufzeit von 330 Tagen beträgt die Glyoxalausbeute 60 Mol% bei einer Ausbeute an Formaldehyd und $CO_x$ von 6,4 und 25 Mol%.

## Patentansprüche

1. Verfahren zur Herstellung von Carbonylverbindungen der Formel

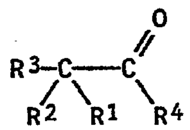 I,

in der $R^1$ ein Wasserstoffatom oder zusammen mit $R^2$ ein Sauerstoffatom, $R^2$ den Rest $OR^5$ oder zusammen mit $R^1$ ein Sauerstoffatom, $R^3$ ein Wasserstoffatom, einen Alkylrest mit 1 bis 8 C-Atomen oder einen Cycolhexyl- oder Cyclopentylrest, $R^4$ ein Wasserstoffatom oder einen Alkylrest mit 1 bis 3 C-Atomen, und $R^5$ einen Alkylrest mit 1 bis 4 C-Atomen, einen Cyclohexyl- oder Cyclopentylrest oder einen Rest der Fromel $-CH_2-CHO$ oder $-CH_2-CH_2-O-CH_2-CHO$ bedeuten, durch Gasphasenoxidation von Alkoholen der Formel

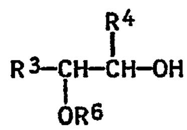 II,

in der $R^3$ und $R^4$ die oben angegebene Bedeutung haben und $R^6$ ein Wasserstoffatom, einen Alkylrest mit 1 bis 4 C-Atomen, einen Cyclohexyl- oder Cyclopentylrest oder einen Rest der Formel $-CH_2-CH_2-OH$ oder $-CH_2-CH_2-O-CH_2-CH_2-OH$ bedeutet, mit einem Sauerstoff enthaltenden Gas in Gegenwart von Kupfer oder Silber enthaltenden Katalysatoren und einer unter den Reaktionsbedingungen flüchtigen Phosphorverbindungen, dadurch gekennzeichnet, daß die Phospormenge (brechnet P), bezogen auf die Gewichtsmenge an dem eingesetzten Alkohol, gringer ist als 0,5 ppm.

2. Verfahren nach Anspruch 1, dadurch gekennzeichnet, daß die Phosphormenge (berechnet P), bezogen auf die Gewichtsmenge an dem eingesetzten Alkohol, 0,05 bis 0,48 ppm beträgt.

3. Verfahren nach Anspruch 1, dadurch gekennzeichnet, daß man als Alkohol der Fromel II eine der Verbindungen der Formeln

$HOCH_2-CH_2OH$, $H_3COCH_2-CH_2OH$, $C_2H_5OCH_2-CH_2OH$,

$H_3C-CH(OH)-CH_2-OH$, $C_2H_5-CH(OH)-CH_2OH$, 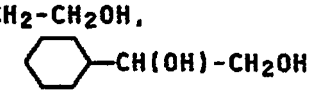,

$HO-(CH_2)_2-O-(CH_2)_2-OH$, 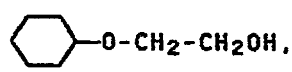, 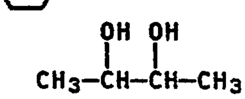

einsetzt.

4. Verfahren nach Anspruch 1, dadurch gekennzeichnet, daß man Glyoxal aus Ethylenglykol herstellt.

5. Verfahren nach Anspruch 1, dadurch gekennzeichnet, daß man die Gasphasenoxidation bei Temperaturen von 225 bis 500°C durchführt.

6. Verfahren nach Anspruch 1, dadurch gekennzeichnet, daß man als flüchtige Phosphorverbindung Ester der Phosphorsäure, der phosphorrigen Säure oder von Phosphornsäuren verwendet.

## Revendications

1. Procédé de préparation de composés carbonylés de formule

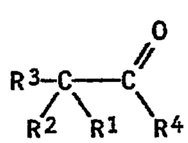 I,

dans laquelle $R^1$ représente un atome d'hydrogène ou, avec $R^2$, un atome d'oxygène, $R^2$ représente un reste $OR^5$ ou, avec $R^1$, un atome d'oxygène, $R^3$ représente un atome d'hydrogène, un reste alkyle à 1 - 8

atomes de carbone ou une reste cyclohexyle ou cyclopentyle, $R^4$ représente un atome d'hydrogène ou un reste alkyle à 1 - 3 atomes de carbone, et $R^5$ représente un reste alkyle à 1 - 4 atomes de carbone, un reste cyclohexyle ou cyclopentyle ou un reste de formule $-CH_2-CHO$ ou $-CH_2-CH_2-O-CH_2-CHO$, par oxydation en phase gazeuse d'alcools de formule

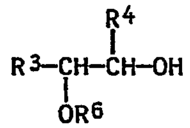

II,

dans laquelle $R^3$ et $R^4$ ont les significations données cidessus et $R^6$ représente un atome d'hydrogène, un reste alkyle à 1 - 4 atomes de carbone, un reste cyclohexyle ou cyclopentyle ou un reste de formule $-CH_2-CH_2-OH$ ou $-CH_2-CH_2-O-CH_2-CH_2-OH$, avec un gaz contenant de l'oxygène en présence de catalyseurs contenant du cuivre ou de l'argent et d'un composé du phosphore volatil dans les conditions de la réaction, caractérisé en ce que la quantité de phosphore (P calculé) est inférieure à 0,5 ppm par rapport à la quantité en poids de l'alcool mis en réaction.

2. Procédé selon la revendication 1, caractérisé en ce que la quantité de phosphore (P calculé) se situe entre 0,05 et 0,48 ppm par rapport à la quantité en poids de l'alcool mis en réaction.

3. Procédé selon la revendication 1, caractérisé en ce qu'on utilise, comme alcool de formule II, l'un des composés de formules

$HOCH_2-CH_2OH$, $H_3COCH_2-CH_2OH$, $C_2H_5OCH_2-CH_2OH$,
$H_3C-CH(OH)-CH_2-OH$, $C_2H_5-CH(OH)-CH_2OH$, 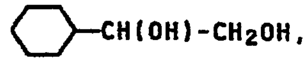,
$HO-(CH_2)_2-O-(CH_2)_2-OH$, 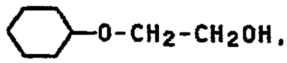, 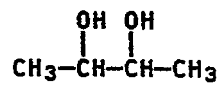

4. Procédé selon la revendication 1, caractérisé en ce qu'on prépare du glyoxal à partir d'éthylèneglycol

5. Procédé selon la revendication 1, caractérisé en ce qu'on conduit l'oxydation en phase gazeuse à des températures de 225 à 500°C.

6. Procédé selon la revendication 1, caractérisé en ce que l'on utilise, comme composé du phosphore volatil, des esters de l'acide phosphorique, de l'acide phosphoreux ou d'acides phosphoniques.

## Claims

1. A process for the preparation of a carbonyl compound of the formula

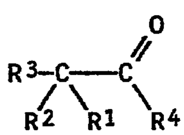

I,

where $R^1$ is hydrogen or, together with $R^2$, is oxygen, $R^2$ is the radical $OR^5$ or, together with $R^1$, is oxygen, $R^3$ is hydrogen, alkyl of 1 to 8 carbon atoms, cyclohexyl or cyclopentyl, $R^4$ is hydrogen or alkyl of 1 to 3 carbon atoms, $R^5$ is alkyl of 1 to 4 carbon atoms, cyclohexyl or cyclopentyl or a radical of the formula $-CH_2-CHO$ or $-CH_2-CH_2-O-CH_2-CHO$, by gas-phase oxidation of an alcohol of the formula

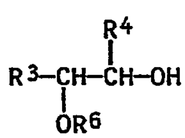

II,

where $R^3$ and $R^4$ have the abovementioned meanings and $R^6$ is hydarogen, alkyl of 1 to 4 carbon atoms, cyclohexyl or cyclopentyl or a radical of the formula $-CH_2-CH_2-HO$ or $-CH_2-CH_2-O-CH_2-CH_2-OH$, with an oxygen-containing gas in the presence of a copper- or silver-containing catalyst and a phosphorus compound wich is volatile under the reaction conditions, wherein the amount of phosphorus (calculated as P) is less than 0,5 ppm, based on the total amount of the alcohol used.

2. A process as claimed in claim 1, wherein the amount of phosphorus (calculated as P) is from 0,05 to 0,48 ppm, based on the weight of the alcohol used.

3. A process as claimed in claim 1, wherein one of the compounds of the fromulae

$HOCH_2-CH_2OH$, $H_3COCH_2-CH_2OH$, $C_2H_5OCH_2-CH_2OH$,

$H_3C-CH(OH)-CH_2-OH$, $C_2H_5-CH(OH)-CH_2OH$, 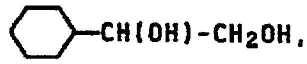,

$HO-(CH_2)_2-O-(CH_2)_2-OH$, 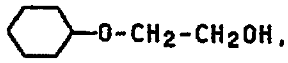, 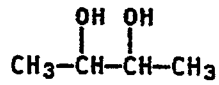

is used as the alcohol of the formula II.

4. A process as claimed in claim 1, wherein glyoxal is prepared from ethylene glycol.

5. A process as claimed in claim 1, wherein the gasphase oxidation is carried out at 225 to 500°C.

6. A process as claimed in claim 1, wherein the volatile phosphorus compound used is an ester of phosphoric acid, of phosphorous acid or of a phosphonic acid.